# EUROPÄISCHE PATENTSCHRIFT

(11) **EP 1 048 656 B1**
(45) Veröffentlichungstag und Bekanntmachung des Hinweises auf die Patenterteilung: **30.11.2005**
(21) Anmeldenummer: 00107441.8
(22) Anmeldetag: 06.04.2000
(51) Int. Cl.: C07D 233/76, C07D 263/44

(54) **Verfahren zur Herstellung von Zwischenstufen von Hydantoinen oder cyclischen Anhydriden einer Aminosäure**
Process for the preparation of intermediates of hydantoins or cyclic anhydrides of an aminoacid
Procédé pour la préparation d'intermédiaires des hydantoins et anhydrides d'une acide aminé

(30) Priorität: 28.04.1999 DE 19919174
(43) Veröffentlichungstag der Anmeldung: 02.11.2000
(73) Patentinhaber: BASF Aktiengesellschaft, 67056 Ludwigshafen (DE)
(72) Erfinder: Slany, Michael, Dr., 67281 Kirchheim (DE); Schulz, Michael, Dr., 67067 Ludwigshafen (DE); Schäfer, Martin, Dr., 67063 Ludwigshafen (DE); Zeller, Edgar, Dr., 68165 Mannheim (DE); Ebel, Klaus, Dr., 68623 Lampertheim (DE)

(56) Entgegenhaltungen:
- EP-A- 0 989 122
- DE-C- 824 635
- DE-C- 19 629 717
- BELLER ET AL.: "Palladium-Catalyzed Synthesis of Substituted Hydabtoins- A New Carbonylation Reaction for the Synthesis of Amino Acid Derivatives" ANGEW. CHEM. INT. ED., Bd. 38, Nr. 10, 17. Mai 1999 (1999-05-17), Seiten 1454-7, XP002188758

## Beschreibung

Die Erfindung betrifft ein Verfahren zur Herstellung von Zwischenstufen von Hydantoinen. Diese Verbindungen sind als Zwischenstufen zur Herstellung von essentiellen Aminosäuren, z.B. Methionin von Bedeutung.

Die katalytische Herstellung von N-Acylglycinderivaten durch Umsetzung eines Aldehyds mit einem Carbonsäureamid und Kohlenmonoxid in Gegenwart einer Übergangsmetallverbindung ist bekannt.

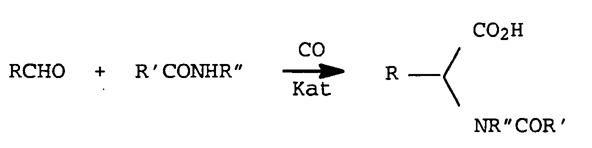

Sie wurde zuerst von Wakamatsu et al. (DE-A- 2115985) beschrieben. Die Umsetzung wurde in Gegenwart von Wasserstoff/CO-Gas mit einem Molverhältnis von CO/H2 = 3:1 durchgeführt. Als Katalysator wurde Cobaltoctacarbonyl in einer Konzentration von 30 mmol Co-Metall je Liter Reaktionsgemisch eingesetzt.

Die EP 0 338 330 beschreibt ein Verfahren zur Herstellung von N-Acylglycinderivaten unter Verwendung eines Gemisches aus einer Palladiumverbindung und einem ionischen Halogenid als Katalysator. Die Reaktion wird bei einem Druck von 120 bar und einer Temperatur von 120°C durchgeführt.

Die DE-19629717 beschreibt für die Amidocarbonylierung von einem Carbonsäureamid (Acetamid) und einem Aldehyd ein Katalysatorsystem folgender Zusammensetzung: PdBr2/PPh3/LiCl/H2SO4. Die Reaktion verläuft bei einem CO-Druck von 60 bar und einer Temperatur von 80°C.

Aus der EP 989 122 ist ein Verfahren zur Herstellung von Imidazolidin-2,4-dionen bekannt, das von Aldehyden und Harnstoffen ausgeht.

Die industrielle Herstellung von DL-Methionin erfolgt derzeit dreistufig ausgehend von Acrolein, an das basenkatalysiert Methylmercaptan zum Methylthiopropionaldehyd (Methional) addiert wird (K. Weissermel, H.-J. Arpe, Industrielle Organische Chemie, 4. Auflage (1994), Seite 312 - 313).

Der Aldehyd wird mit NaCN und Ammoniumhydrogencarbonat in wässriger Lösung bei 90°C zu einem Hydantoin umgesetzt. Dieses wird in der letzten Stufe mit NaOH unter Druck bei 180°C und nach Ansäuern mit H₂SO₄ in das freie DL-Methionin überführt.

Der vorliegenden Erfindung lag die Aufgabe zugrunde, ausgehend von leicht zugänglichen Ausgangsverbindungen eine einfache Synthese von Hydantoinen, bzw. deren Derivanten, oder cyclischen Anhydriden von Aminosäuren zu entwickeln, die potentielle Vorstufen von essentiellen Aminosäuren, besonders Methionin darstellen.

Diese Aufgabe wird gelöst durch ein Verfahren zur Herstellung von Verbindungen der Formel 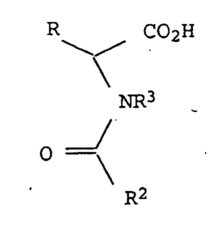 in der R Alkyl oder Aryl, jeweils gegebenenfalls substituiert, R³ Wasserstoff, Alkyl oder Aryl, jeweils gegebenenfalls substituiert, und in der R² -OR' mit R' Alkyl oder Aryl, insbesondere C₁-C₆-Alkyl oder Phenyl, oder -O^{⊖}NH^{⊕}₄ bedeutet,
dadurch gekennzeichnet, daß ein Aldehyd R-CHO mit CO und einer Verbindung der Formel II 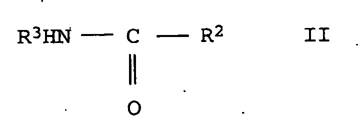 in der R² -OR' mit R' Alkyl oder Aryl, insbesondere C₁-C₆-Alkyl oder Phenyl, oder -O^{⊖}NH^{⊕}₄ bedeutet,
in Gegenwart eines Übergangsmetallkatalysators umgesetzt wird.

Bevorzugt bedeutet R C₁-C₆-Alkyl, wobei der Alkylrest eine funktionelle Gruppe, z.B. -OCH₃, -S-CH₃. -NH₂, tragen kann, insbesondere -CH₂Ph, -CH₂CH(CH₃)₂, -CH(CH₃) -CH₂-CH_{3'} -CH₂-CH₂-S-CH₃, -(CH₂)₄-NH₂ oder Phenyl.

Das erfindungsgemäße Verfahren kann schematisch wie folgt dargestellt werden:

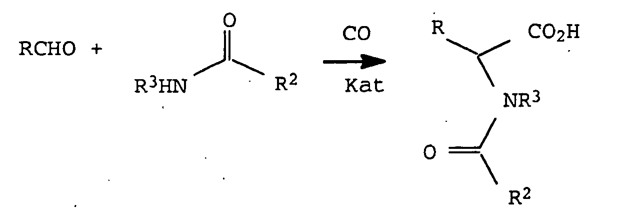

Dieses Verfahren hat den Vorteil, daß man mit den industriell in großem Maße verfügbaren und auch billigen Rohstoffen und CO ausgehend von einem Aldehyd ein Zwischenprodukt für Hydantoin herstellen kann und des weitern kein Salz bei der Synthese anfällt, welches entsorgt werden muß. Die Synthese läßt sich als Eintopfverfahren durchführen.

Als Katalysatoren kommen die an sich bekannten Übergangsmetallkatalysatoren der Metalle Fe, Co, Ni, Ru, Rh, Pd, Os, Ir oder Pt in Frage.

Als Katalysator wird bevorzugt ein Gemisch aus einer Palladiumverbindung, einem ionischen Halogenid und einer Säure eingesetzt, so daß für den Gesamtprozeß Umsätze von 100 % des Amids bei Selektivitäten von bis zu 98 % zum Hydantoin erreicht werden.

Als Palladiumverbindungen können Palladium (II)-Verbindungen, Pd (0)-Verbindungen oder Palladiumphosphankomplexe verwendet werden. Beispiele für Pd(II)-Verbindungen sind Palladiumacetate, -halogenide, -nitrite, -carbonate, -ketonate, -acetylacetonate sowie Allylpalladiumverbindungen. Besonders bevorzugte Vertreter sind PdBr₂, PdCl₂, Li₂PdBr₄, Li₂PdCl₄ und Pd(OAc)₂.

Beispiele für Pd(0)-Verbindungen sind Palladiumphosphin- und Palladiumolefinkomplexe. Besonders bevorzugte Vertreter sind Palladiumbenzylidenkomplexe und Pd(PPh₃)₄.

Beim Einsatz von Palladiumphosphinkomplexen haben sich besonders Bisphosphinpalladium(II)-Komplexe bewährt. Die Komplexe können als solche eingesetzt werden oder in der Reaktionsmischung aus einer Palladium(II)-Verbindung, wie z.B. PdBr₂, PdCl₂ oder Pd(OAc)₂ unter Zusatz von Phosphanen, wie z.B. Triphenylphosphan, Tritolylphosphan, Bis(diphenylphosphino)ethan, 1,3-Bis(diphenylphosphino)propan, 1,4-Bis(diphenyphosphino)butan.

Besonders bevorzugt ist Bistriphenylphosphinopalladium(II)bromid oder -chlorid.

Durch Verwendung von Olefinen mit einem oder mehreren Chiralitätzentren ist es möglich, in der Reaktion zu isomerenreinen oder mit einem Enantiomer angereicherten Produkten zu gelangen.

Die Menge an eingesetzter Palladiumverbindung ist nicht besonders kritisch. Für das erfindungsgemäße Verfahren hat sich gezeigt, daß eine Menge von 0,0001 bis 5 mol-% Palladiumverbindung (berechnet auf Palladiummetall), insbesondere von 0,05 bis 2 mol-%, bezogen auf das Amid, ausreichend ist.

Als ionisches Halogenid können z.B. Phosphoniumbromide und Phosphoniumiodide, z.B. Tetrabutylphosphoniumbromid bzw. Tetrabutylphosphoniumiodid, sowie Ammonium, Lithium, Natrium, Kaliumchlorid, -bromid und -iodid verwendet werden. Bevorzugte Halogenide sind Bromide und Chloride. Vorzugsweise wird das ionische Halogenid in einer Menge von 1 bis 50 mol%, insbesondere von 2 bis 40 mol-%, bezogen auf das Amid eingesetzt.

Als Säuren können organische und anorganische Verbindungen mit einem pKa<5 (relativ zu Wasser) verwendet werden. So können neben organischen Säuren, wie p-Toluolsulfonsäure, Hexafluorpropansäure oder Trifluoressigsäure und anorganische Säuren, wie Schwefelsäure oder Phosphorsäure, auch Ionentauscherharze, wie Amberlyst oder Nafion, verwendet werden. Schwefelsäure ist besonders bevorzugt.

Zweckmäßigerweise wird die Säure in einer Menge von 0,1 bis 20 mol-%, insbesondere von 0,5 bis 5 mol-%, bezogen auf das Amid, eingesetzt.

Als Lösungsmittels sind dipolare aprotische bevorzugt einsetzbar. Beispiele hierfür sind: Sulphoxide und Sulphone, zum Beispiel Dimethylsulphoxid, Diisopropylsulphon oder Tetrahydrothiophen-2,2-dioxid, 2-Methylsulfolan, 3-Methylsulfolan, 2-Methyl-4-butylsulfolan; Ester wie Methylacetat und Butyrolacton; Ketone wie Aceton oder Methylisobutylketon; Ether wie Tetrahydrofuran, Anisol, 2,5,8-Trioxanonan, Dioxan, Diphenylether und Diisopropylether, Ethylenglykoldimethylether; Amide wie Dimethylacetamid, DMF und N-Methylpyrrolidon; Nitrile wie Acetonitril und Carbonsäuren.

Die Reaktion wird im allgemeinen bei Drücken von 0,1 bis 200 bar, vorzugsweise von 2 bis 100 bar, und bei Temperaturen von 20 bis 200°C, vorzugsweise bei 50 bis 150°C, durchgeführt.

Ein Syntheseweg zur Herstellung von Zwischenprodukten von Aminosäuren durch Amidocarbonylierung besteht in der Umsetzung von Urethan mit Aldehyden in Gegenwart von CO und eines Übergangsmetallkatalysators. 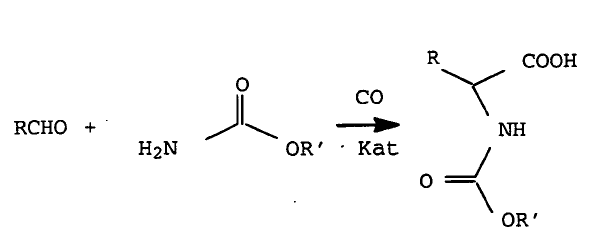 R = Alkyl, besonders CH₂Ph, CH₂CH(CH₃)₂, CH(CH₃)CH₂CH₃, CH(CH₃)₂, funktionalisierte Alkylreste, besonders CH₂-CH₂-SMe, (CH₂)₄NH₂, Aryl
R' = Alkyl, Aryl.

Das Verfahren hat den Vorteil, daß man mit den industriell in großem Maße verfügbaren und auch billigen Rohstoffen Urethan und CO ausgehend von einem Aldehyden eine Aminosäure herstellen kann und des weitern kein Salz bei der Synthese anfällt, welches entsorgt werden muß.

Überraschend wurde gefunden, daß bei Umsetzung von Urethan mit Aldehyden in Gegenwart von Übergangsmetallkatalysatoren die Amidocarbonylierung zu Aminosäuren gelingt.

Ein Zweiter Syntheseweg zur Herstellung von Hydantoinen durch Amidocarbonylierung besteht in der Umsetzung von Ammnoniumcarbamidat (Vorstufe von Harnstoff) mit Aldehyden in Gegenwart von Co und eines Übergangsmetallkatalysators. 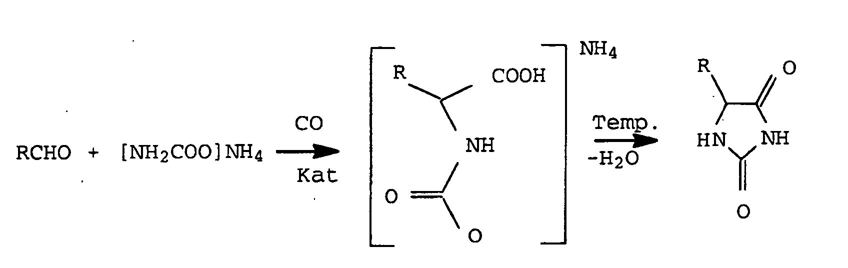
R = Alkyl, besonders CH₂Ph, CH₂CH(CH₃)₂, CH(CH₃)CH₂CH₃, CH(CH₃)₂, funktionalisierte Alkylreste, besonders CH₂-CH₂-SMe, (CH₂)₄NH₂, Aryl

Das Verfahren hat den Vorteil, daß man mit den industriell in großem Maße verfügbaren und auch billigen Rohstoffen Ammoniumcarbamidat (Harnstoff-Vorstufe) und CO ausgehend von einem Aldehyden ein Hydantoin unter Wasserabspaltung herstellen kann und des weitern kein Salz bei der Synthese anfällt, welches entsorgt werden muß.

Überraschend wurde gefunden, daß bei Umsetzung von Ammoniumcarbamidat (Harnstoff-Vorstufe) mit Aldehyden in Gegenwart von Übergangsmetallkatalysatoren die Amidocarbonylierung und in einem weiteren Schritt die Umsetzung zu Hydantoinen unter Wasserabspaltung gelingt.

### Beispiel (nicht erfindungs gemäß)

### Methioninsynthese über Amidocarbonylierung von Harnstoff

### 1) Amidocarbonylierung von Methional mit Harnstoff

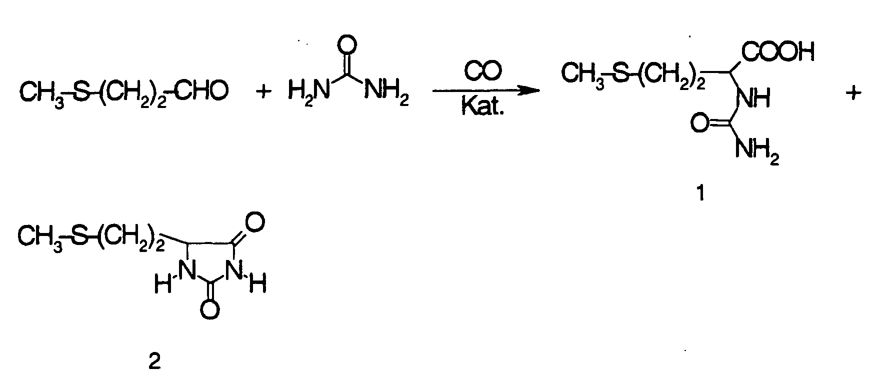

### Beispiel zur Amidocarbonylierung:

67 mg PdBr₂ und 132 mg PPh₃ wurden in 75 ml NMP gelöst und 1 Stunde gerührt. Danach wurden 2,6 g LiBr, 100 mg H₂SO₄ (konz.), 15 g o-Ameisensäuretriethylester, 6,1 g Harnstoff und 10,4 g Methional zur Lösung gegeben und in einen 270-ml-Autoklaven überführt. Der Autoklav wurde bei einem CO-Druck von 60 bar auf 100°C aufgeheizt und 10 Stunden betrieben. Nach Abkühlen und Entspannen des Autoklaven wurde der Flüssigaustrag GC-analytisch untersucht.

| | |
|---|---|
| Umsatz Methional | 95 % |
| Selektivität Hydantoin 2 | 86 % |
| Selektivität N-Carbamoylsäure 1 | 12 % |

### 2) Umsetzung des Hydantoins zu D,L-Methionin

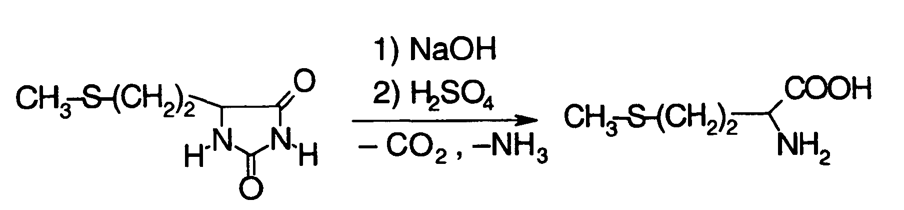

Technisch durchgeführter Prozeß (K. Weissermel, H.-J. Arpe, " Industrielle Organische Chemie", 4. Auflage)

## Patentansprüche

1. Verfahren zur Herstellung von Verbindungen der folgenden Formel 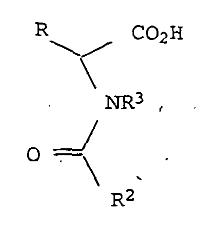 in der R Alkyl oder Aryl, jeweils gegebenenfalls substituiert, R³ Wasserstoff, Alkyl oder Aryl, jeweils gegebenenfalls substituiert, und in der R² -OR' mit R' Alkyl oder Aryl, oder -O^{⊖}NH^{⊕}₄ bedeutet,
**dadurch gekennzeichnet, daß** ein Aldehyd R-CHO mit CO und einer Verbindung der Formel II 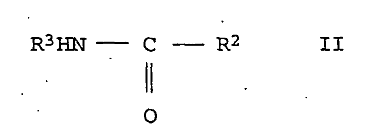 in Gegenwart eines Übergangsmetallkatalysators umgesetzt wird.

2. Verfahren nach Anspruch 1, **dadurch gekennzeichnet, daß** R -CH₂Ph, -CH₂CH(CH₃)₂, -CH(CH₃)-CH₂-CH_{3'} -CH₂-CH₂-S-CH₃, -(CH₂)₄-NH₂ oder Phenyl ist.

## Claims

1. A process for preparing compounds of the following formula 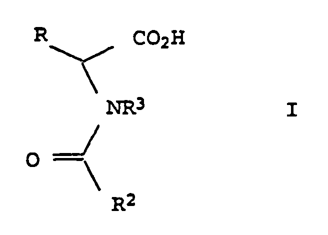 in which R is alkyl or aryl, in each case unsubstituted or substituted, R³ is hydrogen, alkyl or aryl, in each case unsubstituted or substituted, and in which R² is -OR' where R' is alkyl or aryl, or is -O^{⊖}pNH^{⊕}₄,
which comprises reacting an aldehyde R-CHO with CO and a compound of the formula II 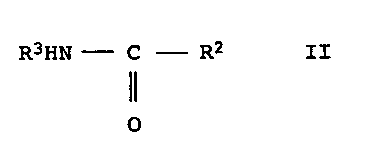 in the presence of a transition metal catalyst.

2. A process as claimed in claim 1, wherein R is -CH₂Ph, -CH₂CH(CH₃)₂, -CH(CH₃)-CH₂-CH₃' -CH₂-CH₂-S-CH₃, -(CH₂)₄-NH₂ or phenyl.

## Revendications

1. Procédé de préparation de composés de la formule suivante : 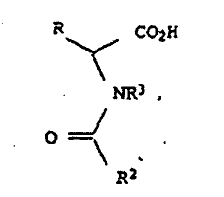 dans laquelle R représente un groupe alkyle ou aryle, chacun éventuellement substitué, R³ représente de l'hydrogène ou un groupe alkyle ou aryle, chacun éventuellement substitué, et R² représente -OR' , où R' est un alkyle ou un aryle, ou -O^{⊖}PNH^{⊕}₄,
**caractérisé en ce qu'**on fait réagir un aldéhyde R-CHO avec CO et un composé de la formule II : 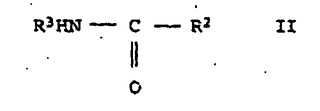 en présence d'un catalyseur à base de métal de transition.

2. Procédé suivant la revendication 1, **caractérisé en ce que** R est un groupe -CH₂Ph, -CH₂CH (CH₃)₂, -CH(CH₃)-CH₂-CH₃, -CH₂-CH₂-S-CH₃, -(CH₂)₄-NH₂ ou phényle.
